# NOUVEAU FASCICULE DE BREVET EUROPEEN

(11) **EP 0 614 874 B2**
(45) Date de publication et mention de la décision concernant l'opposition: **23.10.2002**
(45) Mention de la délivrance du brevet: 27.01.1999
(21) Numéro de dépôt: 94400507.3
(22) Date de dépôt: 08.03.1994
(51) Int. Cl.: C07C 67/11, C07C 69/54

(54) **Procédé de fabrication d'esters halogénés d'acides carboxyliques ou dicarboxyliques**
Verfahren zur Herstellung von hologenierten Estern von Carbonsäuren oder Dicarbonsäuren
Process for the preparation of halogenated esters of carboxylic or dicarboxylic acids

(30) Priorité: 09.03.1993 FR 9302689; 30.07.1993 FR 9309435
(43) Date de publication de la demande: 14.09.1994
(73) Titulaire: Atofina, 92800 Puteaux (FR)
(72) Inventeur: Drivon, Gilles, F-69850 Saint-Martin en Haut (FR); Gillet, Jean-Philippe, F-69530 Brignais (FR); Suc, Sophie, F-69630 Chaponost (FR)
(74) Mandataire: Chaillot, Geneviève

(56) Documents cités:
- EP-A- 0 101 526
- DE-C- 2 660 331
- GB-A- 2 117 376
- GB-A- 2 123 408
- JP-A- 61 217 223
- US-A- 2 830 078
- PATENT ABSTRACTS OF JAPAN vol. 10, no. 235 (C-366) (2291) 14 Août 1986 & JP-A-61 069 742 (MITSUBISHI METAL CORP.)
- Industrial Chemical Magazine, vol. 69(4), 1966, pp. 641-643, S. Yoneda et al

## Description

La présente invention concerne un nouveau Procédé de fabrication d'un composé représenté par la formule générale (Ia) : 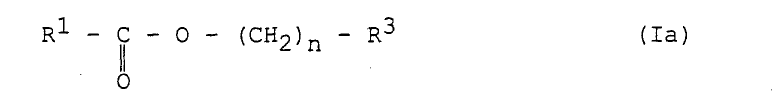 où :
- R¹ représente un radical alcényle en C₂-C₆ ;
- R³ représente un radical alkyle perfluoré en C₁-C₁₀ ;
- 0 < n ≤ 4,
   caractérisé en ce que l'on fait réagir dans un solvant aprotique polaire, sur un sel d'acide carboxylique de formule (IIa) : 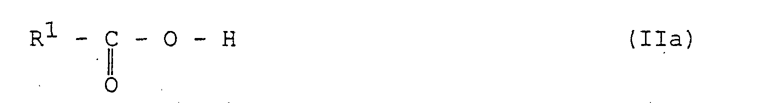 où le radical R¹ est tel que défini ci-dessus, un composé de formule (III) :

   R³- (CH₂)ₙ - X (III)
dans laquelle :
- R³ et n sont tels que définis ci-dessus ; et
- X représente un halogène,
ladite réaction étant effectuée entre 0,10 et 2 bars, avec introduction en continu dans un réacteur soit du composé de formule (III) dans une suspension, préalablement introduite dans le réacteur, du sel de l'acide de formule (IIa) dans le solvant aprotique polaire, soit à la fois du composé (III) et d'une suspension du sel de l'acide (IIa) dans le solvant aprotique polaire, le produit formé (Ia) étant récupéré en continu au fur et à mesure de sa formation.

Ces esters trouvont leurs applications dans les domaines suivants:
peintures anti-poussière ou anti-salissure pour différents supports comme les revêtements extérieurs et le mobilier métallique ; fibres optiques ; lentilles de contact lithographie ; électrophotographie ; matériaux résistants à la chaleur ; résines dentaires.

Parle brevet français FR-B-2 583 414, on connaît un procédé de préparation de ces (méth)acrylates d'alkyle fluorés, de formule (Ia), procédé suivant lequel on fait réagir un anhydride (méth)acrylique de formule (a) : 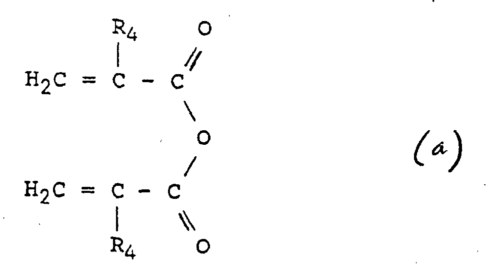 où R₄ est methyle ou hydrogène, avec le 2,2,2-trifluoroéthanol, en présence d'un inhibiteur de polymérisation et d'un catalyseur acide, le rapport molaire de l'anhydride par rapport à l'alcool étant compris entre 0,5 et 5, puis on sépare le composé (Ia) résultant.

Les composés de formule (Ia), preparés de cette façon, sont des produits onéreux, en raison du coût élevé du 2,2,2-trifluoroéthanol et des problèmes de séparation. On a donc recherché une nouvelle voie de synthèse, plus économique, à partir de produits disponibles en quantité industrielle.

On a maintenant réussi à mettre au point une nouvelle voie d'accès aux composés de formule (Ia). Il n'était pas évident qu'une telle réaction puisse avoir lieu, d'une part, à cause de la faible réactivité du composé (III) et, d'autre part, à cause des risques de polymérisation dans le cas des acides insaturés.

On a cependant découvert que, de façon surprenante, cette réaction pouvait être effectuée avantageusement à pression atmosphérique normale ou au voisinage de la pression atmosphérique normale, par exemple sous une pression comprise entre environ 0,10 bar et 2 bar,
- soit par introduction continue dans le réacteur du composé (III) dans une suspension, préalablement introduite dans le réacteur, du sel de l'acide (IIa) dans le solvant précité,
- soit par introduction continue dans le réacteur du composé (III) et de la suspension de sel dans le solvant, le produit formé (Ia) étant, dans les deux modes de réalisation, récupéré en continu au fur et à mesure de sa formation.

Les avantages d'une opération conduite à la pression atmosphérique résident dans le fait qu'il n'est pas nécessaire de disposer d'un matériel résistant à la pression et que le composé (la) est récupéré par distillation continue au fur et à mesure de sa formation. Ceci limite les réactions parasites d'hydrolyse et de dimérisation, ce qui simplifie d'autant sa purification finale. D'autre part, ce procédé assure toute sécurité dans la mesure où l'on opère à pression atmosphérique normale ou voisine de la normale et où l'on diminue fortement les risques de polymérisation dans le réacteur.

Parmi les sels des acides de formule (IIa), on peut citer, entre autres, ceux des acides dans lesquels :
- R¹ représente un radical alcényle en C₂-C₆.

Comme sels des acides (IIa), on utilise par exemple un sel de métal alcalin, tel que Na, K, Rb, Cs, ou un sel d'un métal alcalino-terreux, tel que Mg, Ca.

Comme composés de formule (III), on peut citer ceux pour lesquels :
- R³ représente un radical alkyle perfluoré en C₁-C₁₀ ; et
- X représente fluor, chlore, brome, iode.

Parmi les composés de formule (III), on peut mentionner notamment :
. le 2-chloro-1,1,1-trifluoroéthane ; le 1,2-dichloro-1,1-difluoroéthane ;
. le 2-bromo-1,1,1-trifluoroéthane ;
. le 2-perfluorohexyl-1-iodoéthane.

On choisit le solvant aprotique polaire par exemple dans le groupe constitué par le sulfolane (tétraméthylène sulfone), le N,N-diméthylformamide, le diméthylsulfoxyde, le N,N-diméthylacétamide, la N-méthylpyrrolidone, la 1,3-diméthyl-2-imidazolidinone, la 1,3-diméthyl-3,4,5,6-tétrahydro-2-pyrimidinone et leurs mélanges. De préférence, on utilise le sulfolane ou la 1,3-diméthyl-2-imidazolidinone.

Pour l'amélioration de la cinétique de la réaction, il est avantageux de conduire cette dernière en présence d'un agent de transfert de phase (éthers couronne, composés polyéthoxylés comme le nonyl phénol oxyéthyléné (commercialisé sous la dénomination d'ANTAROX CO 990) ou la Tris[2-(2-méthoxyéthoxy)éthyl]amine, etc.), à raison de 0,1 à 5% molaire par rapport au sel de l'acide de formule (IIa) ou (llb) engagé.

Par ailleurs, on utilise avantageusement un rapport molaire du composé de formule (III) sur le sel de l'acide de formule (IIa) compris entre environ 0,2 et 10, de préférence entre environ 1 et 7. En outre, la concentration du sel de l'acide de formule (IIa) par rapport au solvant est avantageusement comprise entre 5 et 50% en poids, de préférence, entre 10 et 40% en poids.

La température de réaction est généralement comprise entre environ 50 et 280°C, de préférence entre environ 120 et 240°C.

De plus, la réaction est réalisée en présence ou non d'un stabilisant, par exemple, la N,N'-diphényl-p-phénylènediamine.

Pour mieux illustrer l'objet de la présente invention, on va en décrire ci-après, à titre indicatif et non limitatif, plusieurs exemples de réalisation. Les pourcentages indiqués dans ces exemples sont en poids, sauf indication contraire. Les abréviations utilisées sont les suivantes :
ATRIFE : acrylate de 2,2,2-trifluoroéthyle
MATRIFE : méthacrylate de 2,2,2-trifluoroéthyle
ACTRIFE : acétate de 2,2,2-trifluoroéthyle
AK : acrylate de potassium
MAK : méthacrylate de potassium
Forane 133a : 2-chloro-1,1,1-trifluoroéthane
DPPD : N,N'-diphényl-p-phénylène diamine (stabilisant)

### Exemple 1 : Préparation du MATRIFE par réaction du MAK avec le Forane 133a à la pression atmosphérique

Dans un réacteur en verre, d'une capacité de 500 cm³, équipé d'un dispositif d'agitation (turbine), d'un dispositif de chauffage et d'un système permettant de réguler la température, on introduit 321 g de sulfolane, 0,08 g de DPPD et 40 g de MAK. On chauffe à 210°C sous agitation, puis on introduit en continu le Forane 133a, sous forme gazeuse, à l'aide d'une canne d'introduction en pied du réacteur, à la pression atmosphérique, pendant 5 heures 30, à un débit moyen d'environ 39 g par heure. Le MATRIFE formé est récupéré au fur et à mesure de sa formation par condensation à 15°C des "évents" gazeux.

On récupère 82,6 g d'un condensat constitué par 50,8 g de MATRIFE, mélangé essentiellement à du Forane 133a. La conversion du MAK est de 99,9% et la sélectivité par rapport au MAK transformé est de 94%.

Tous ces résultats sont déterminés par analyse du milieu réactionnel et des condensats, par chromatographie en phase gazeuse et par potentiométrie.

### Exemples 2 à 4

L'Exemple 1 est répété, avec des conditions différentes, comme rapporté dans le Tableau 1 suivant. Les résultats quant à la conversion et à la sélectivité sont également indiqués dans le Tableau 1.

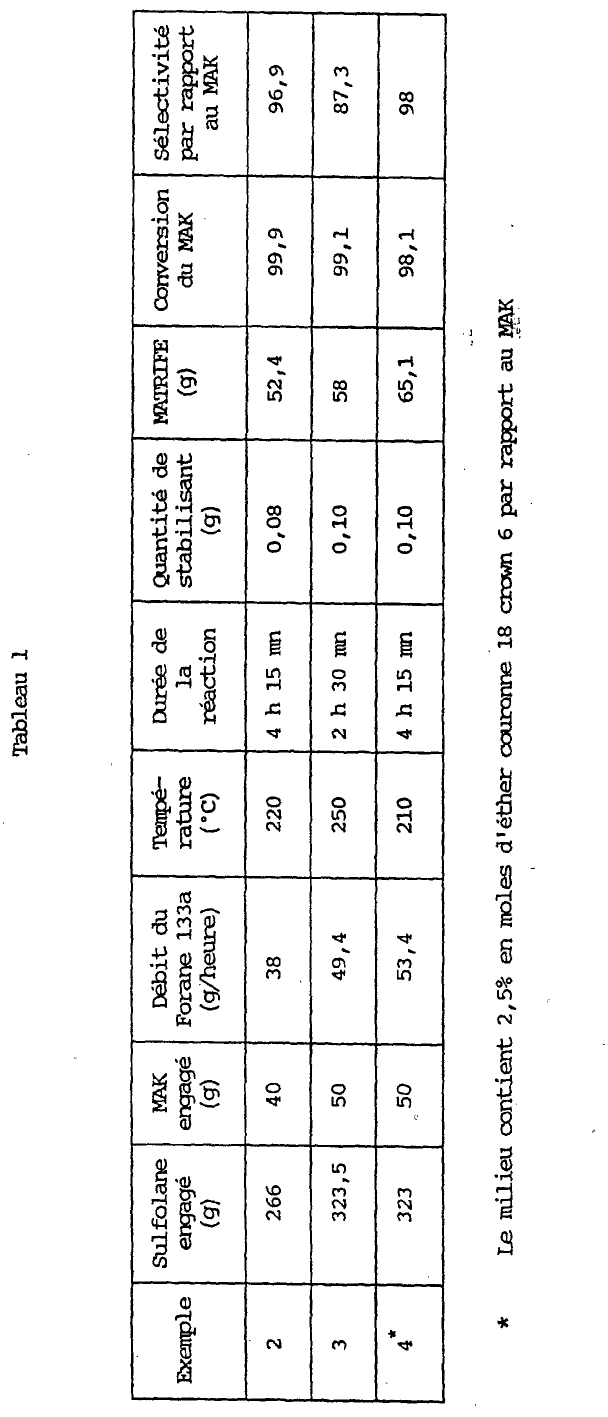

## Revendications

1. Procédé de fabrication d'un composé représenté par la formule générale (Ia) : 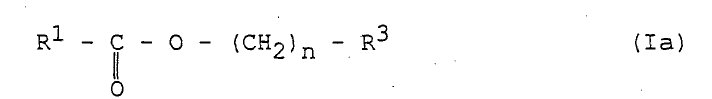 où :
- R¹ représente un radical alcényle en C₂-C₆ ;
- R³ représente un radical alkyle perfluoré en C₁-C₁₀ ;
- 0 < n ≤ 4,
**caractérisé en ce que** l'on fait réagir dans un solvant aprotique polaire, sur un sel d'acide carboxylique de formule (IIa) : 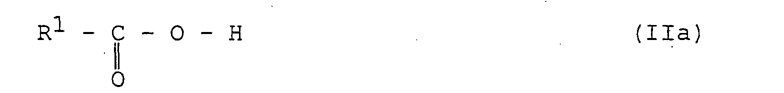 où le radical R¹ est tel que défini ci-dessus, un composé de formule (III) :
R³- (CH₂)ₙ - X (III)
dans laquelle :
- R³ et n sont tels que définis ci-dessus ; et
- X représente un halogène,
ladite réaction étant effectuée entre 0,10 et 2 bars, avec introduction en continu dans un réacteur soit du composé de formule (III) dans une suspension, préalablement introduite dans le réacteur, du sel de l'acide de formule (IIa) dans le solvant aprotique polaire, soit à la fois du composé (III) et d'une suspension du sel de l'acide (IIa) dans le solvant aprotique polaire, le produit formé (Ia) étant récupéré en continu au fur et à mesure de sa formation.

2. Procédé suivant la revendication 1, dans lequel le composé (Ia) a la formule (A) : 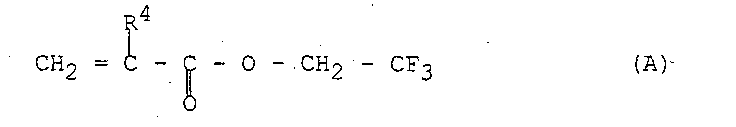 R⁴ étant un radical méthyle ou un atome d'hydrogène.

3. Procédé selon l'une des revendications 1 et 2, **caractérisé par le fait que** le produit foxmé est récupéré par distillation continue au fur et à mesure de sa formation.

4. Procédé suivant l'une des revendications 1 à 3, **caractérisé en ce que** l'on conduit la réaction à une température de 50 à 280°C.

5. Procédé suivant l'une des revendications 1 à 4, **caractérisé en ce que** l'on utilise, comme sel de l'acide carboxylique de formule (IIa), un sel de métal alcalin ou alcalino-terreux.

6. Procédé suivant l'une des revendications 1 à 5, **caractérisé en ce que** l'on choisit le solvant aprotique polaire parmi le sulfolane, le N,N-diméthylformamide, le diméthylsulfoxyde, le N,N-diméthylacétamide, la N-méthylpyrrolidinone, la 1,3-diméthyl-2-imidazolidone, la 1,3-diméthyl-3,4,5,6-tétrahydro-2-pyrimidinone et leurs mélanges.

7. Procédé suivant l'une des revendications 1 à 6, **caractérisé en ce que** l'on conduit la réaction en présence d'au moins un agent de transfert de phase, à raison de 0,1 à 5% molaire par rapport au sel de l'acide carboxylique de formule (IIa) engagé.

8. Procédé suivant l'une des revendications 1 à 7, **caractérisé en ce que** l'on conduit la réaction avec un rapport molaire du composé de formule (III) sur le sel de l'acide carboxylique de formule (IIa) compris entre 0,2 et 10.

9. "Procédé suivant l'une des revendications 1 à 8, **caractérisé en ce que** l'on opère à une concentration du sel de l'acide carboxylique (IIa) comprise entre 5 et 50% en poids par rapport au solvant.

10. Procédé suivant l'une des revendications 1 à 9, **caractérisé en ce que** l'on conduit la réaction en présence d'au moins un stabilisant, tel que la N',N'-diphényl-p-phénylènediamine.

## Patentansprüche

1. Verfahren zur Herstellung einer Verbindung der allgemeinen Formel (Ia) 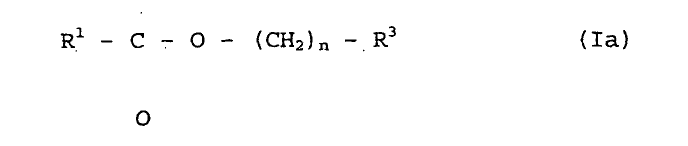 in der bedeuten :
- R¹ C₂₋₆-Alkenyl ;
- R³ perfluoriertes C₁₋₁₀-Alkyl,
- 0 < n
**dadurch gekennzeichnet, daß** in einem polaren aprotischen Lösungsmittel ein Salz einer Carbonsäure der Formel (IIa) 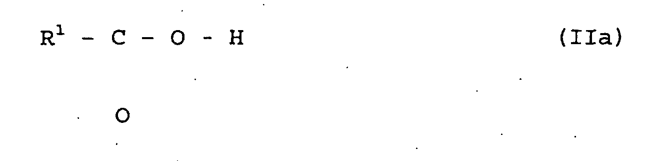 worin R¹ wie weiter oben definiert ist, mit einer Verbindung der Formel (III)
R³- (CH₂)n- X (III)
umgesetzt wird, in der bedeuten :
- R³ und n dasselbe wie weiter oben angegeben und
- X ein Halogen,
wobei die Reaktion bei 0,10 bis 2 bar, durch kontinuierliches Einbringen in den Reaktor entweder der Verbindung der Formel III in eine Suspension, die zuvor in dem Reaktor vorgelegt wurde, des Salzes der Säure der Formel (IIa) in dem polaren aprotischen Lösungsmittel, oder der Verbindung (III) und der Suspension des Salzes in dem aprotischen Lösungsmittel, wobei das gebildete Produkt kontinuierlich in dem Maße, wie es gebildet wird, gewonnen wird.

2. Verfahren nach Anspruch 1, in denen der Verbindung (Ia) hat der Formel (A) : 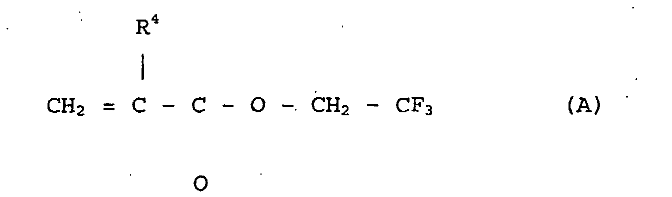 in der R⁴ Methyl oder Wasserstoff bedeutet.

3. Verfahren nach einem der Ansprüche 1 und 2, **dadurch gekennzeichnet, daß** das gebildete Produkt durch kontinuierliche Distillation in dem Maße, wie es gebildet wird, gewonnen wird.

4. Verfahren nach einem der Ansprüche 1 bis 5, **dadurch gekennzeichnet, daß** die Umsetzung bei einer Temperatur von 50 bis 280 °C durchgeführt wird.

5. Verfahren nach einem der Ansprüche 1 bis 6, **dadurch gekennzeichnet, daß** es sich bei dem verwendeten Salz der Carbonsäure der Formel (IIa) um ein Alkalimetallsalz oder Erdalkalimetallsalz handelt.

6. Verfahren nach einem der Ansprüche 1 bis 5, **dadurch gekennzeichnet, daß** das polare aprotische Lösungsmittel unter Sulfolan, N,N-Dimethylacetamid, Di-methylsulfoxid, N,N-Dimethylacetamid, N-Methylpyrrolidon, 1,3-Dimethyl-2-imidazolidinon, 1,3-Dimethyl-3,4,5,6-tetrahydro-2-pyrimidinon und deren Gemischen ausgewählt wird.

7. Verfahren nach einem der Ansprüche 1 bis 6, **dadurch gekennzeichnet, daß** die Umsetzung in Gegenwart mindestens eines Phasentransfermittels durchgeführt wird, das in einer Menge von 0,1 bis 5 mol-%, bezogen auf das eingestzte Salz der Carbonsäure der Formel (IIa) verwendet wird.

8. Verfahren nach einem der Ansprüche 1 bis 7, **dadurch gekennzeichnet, daß** die.Umsetzung entweder bei einem Molverhältnis der Verbindung der Formel (III) zum Salz der Carbonsäure der Formel (IIa) von 0,2 bis 10.

9. Verfahren nach einem der Ansprüche 1 bis 8, **dadurch gekennzeichnet, daß** die Reaktion bei einer Konzentration des Salzes der Carbonsäure (IIa) von 5 bis 50 Gew.-%, bezogen auf das Lösungsmittel, durchgeführt wird.

10. Verfahren nach einem der Ansprüche 1 bis 9, **dadurch. gekennzeichnet, daß** die Umsetzung in Gegenwart mindestens eines Stabilisierungsmittels wie N,N'-Diphenyl-p-phenylendiamin durchgeführt wird.

## Claims

1. Process for the manufacture of a compound represented by the general formula (Ia) : 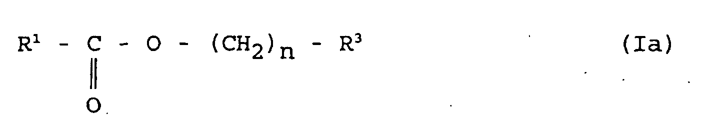 in which :
- R¹ represents a C₂-C₆ alkenyl radical ;
- R³ represents a C₁-C₁₀ perfluoro radical ;
- 0 < n ≤ 4 ;
**characterized in that**, in a polar aprotic solvent, a salt of a carboxylic acid of formula (IIa) : 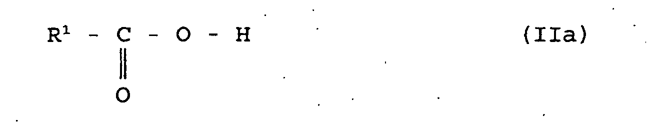 in which the radical R¹ is as defined above, is reacted with a compound of formula (III) :
R³- (CH₂)ₙ- X (III)
in which :
- R³ and n are as defined above ; and
- X represents a halogen,
said reaction being carried out under a pressure between 0.10 bar and 2 bars, with continuous introduction into a reactor either of the compound of formula (III) into a suspension, introduced into the reactor beforehand, of the salt of the acid of formula (IIa) in the polar aprotic solvent, or of both the compound (III) and a suspension of the salt of the acid (IIa) in the polar aprotic solvent, the product formed (Ia) being recovered continuously as it is formed.

2. Process according to claim 1, wherein the compound (Ia) has the formula (A) : 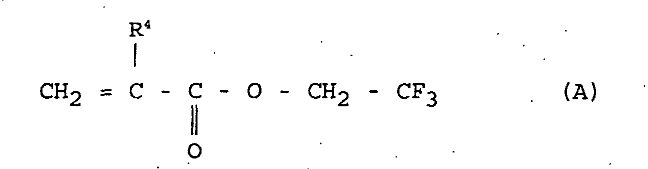 R⁴ being a methyl radical or an hydrogen atom.

3. Process according to anyone of claims 1 and 2, **characterized in that** the product formed is recovered by continuous distillation as it is formed.

4. Process according to anyone of Claims 1 to 3, **characterized in that** the reaction is carried out at a temperature of 50 to 280°C.

5. Process according to anyone of Claims 1 to 4, **characterized in that** an alkali metal salt or alkaline-earth metal salt is used as salt of the carboxylic acid of formula (IIa).

6. Process according to anyone of Claims 1 to 5, **characterized in that** the polar aprotic solvent is chosen from sulpholane, N,N-dimethylformamide, dimethyl sulphoxide, N,N-dimethylacetamide, N-methylpyrrolidone, 1,3-dimethyl-2-imidazolidinone, 1,3-dimethyl-3,4,5,6-tetrahydro-2-pyrimidinone and their mixtures.

7. Process according to anyone of Claims 1 to 6, **characterized in that** the reaction is carried out in the presence of at least one phase transfer agent, in an amount of 0.1 to 5 molar % relative to the salt of the carboxylic acid of formula (IIa) employed.

8. Process according to anyone of Claims 1 to 7, **characterized in that** the reaction is carried out with a molar ratio of the compound of formula (III) to the salt of the carboxylic acid of formula (IIa) between 0.2 and 10.

9. Process according to anyone of Claims 1 to 8, **characterized by** the fact that the procedure is performed at a concentration of the salt of the carboxylic acid (IIa) between 5 and 50% by weight relative to the solvent.

10. Process according to anyone of Claims 1 to 9, **characterized in that** the reaction is carried out in the presence of at least one stabilizing agent, such as N,N'-diphenyl-p-phenylenediamine.
